# EUROPEAN PATENT APPLICATION

(11) **EP 4 316 365 A1**
(43) Date of publication of application: **07.02.2024**
(21) Application number: 22188335.8
(22) Date of filing: 02.08.2022
(51) Int. Cl.: A61B 5/026, A61B 5/0507

(54) **DETERMINING VELOCITY FROM SPECKLE IMAGES**

(71) Applicant: Nokia Technologies Oy, 02610 Espoo (FI)
(72) Inventor: WRIGHT, Christopher, London, E15 4AD (GB); MCDONALD, Rory, Cambridge, CB22 4PN (GB)
(74) Representative: Nokia EPO representatives

(57) **Abstract**

Examples of the disclosure relate to determining velocities of objects that generate a speckle pattern when illuminated. In examples of the disclosure a coherent light source is controlled to illuminate a sample to cause generation of speckle patterns in light backscattered from the sample. An image stabilization system is controlled to configure an imaging device in a plurality of different positions. The imaging device is controlled to capture images of the sample at the plurality of different positions wherein the images comprise the speckle patterns. The speckle patterns in images captured with the imaging device at different positions are compared to identify two images with a level of correlation of the speckle pattern above a threshold. A velocity of one or more objects within the sample is determined based on the respective positions of the imaging device corresponding to the two images with a level of correlation of the speckle pattern above a threshold.

## Description

### TECHNOLOGICAL FIELD

Examples of the disclosure relate to determining velocity from speckle images. Some relate to determining velocity from speckle images using LiDAR (Light Detection and Ranging) systems.

### BACKGROUND

Speckle images can be obtained by illuminating samples such as biological tissue with coherent light. The light backscattered from different positions in the sample can be detected by an image sensor or other suitable means to generate an image. Scatterers within the sample cause different intensities in different parts of the images and so create a speckle image. The movement of these scatterers can be monitored by monitoring changes of the respective areas of varying intensity in the speckle image.

### BRIEF SUMMARY

According to various, but not necessarily all, examples of the disclosure there may be provided an apparatus comprising means for:
controlling a coherent light source to illuminate a sample to cause generation of speckle patterns in light backscattered from the sample;
controlling an image stabilization system to configure an imaging device in a plurality of different positions;
controlling the imaging device to capture images of the sample at the plurality of different positions wherein the images comprise the speckle patterns;
comparing the speckle patterns in images captured with the imaging device at different positions to identify two images with a level of correlation of the speckle pattern above a threshold; and
determining a velocity of one or more objects within the sample based on the respective positions of the imaging device corresponding to the two images with a level of correlation of the speckle pattern above a threshold.

The apparatus may comprise a LiDAR system and the images may comprise the speckle patterns are LiDAR images.

The coherent light source may be configured to project a dot pattern onto the sample.

The coherent light source may be configured to use infrared light to illuminate the sample.

The sample may comprise a biological sample.

The one or more objects whose velocity is determined may comprise blood within the biological sample.

The means may be for obtaining information relating to a pulse and adjusting the movement of the imaging device to account for the pulse.

The different positions of the imaging device may comprise a plurality of different positions relative to the sample.

The image stabilization system may be controlled such that the positions of the imaging device are restricted to take into account a known direction of movement of the one or more objects.

The image stabilization system may be configured to move an image sensor and an optical arrangement of the imaging device.

The level of correlation may be compared for a subset of the image.

According to various, but not necessarily all, examples of the disclosure there may be provided a device comprising an apparatus as described herein wherein the device is at least one of: a telephone, a camera, a computing device, a teleconferencing device, a virtual reality device, an augmented reality device, a headset.

According to various, but not necessarily all, examples of the disclosure there may be provided a method comprising:
controlling a coherent light source to illuminate a sample to cause generation of speckle patterns in light backscattered from the sample;
controlling an image stabilization system to configure an imaging device in a plurality of different positions;
controlling the imaging device to capture images of the sample at the plurality of different positions wherein the images comprise the speckle patterns;
comparing the speckle patterns in images captured with the imaging device at different positions to identify two images with a level of correlation of the speckle pattern above a threshold; and
determining a velocity of one or more objects within the sample based on the respective positions of the imaging device corresponding to the two images with a level of correlation of the speckle pattern above a threshold.

According to various, but not necessarily all, examples of the disclosure there may be provided a computer program comprising computer program instructions that, when executed by processing circuitry, cause:
controlling a coherent light source to illuminate a sample to cause generation of speckle patterns in light backscattered from the sample;
controlling an image stabilization system to configure an imaging device in a plurality of different positions;
controlling the imaging device to capture images of the sample at the plurality of different positions wherein the images comprise the speckle patterns;
comparing the speckle patterns in images captured with the imaging device at different positions to identify two images with a level of correlation of the speckle pattern above a threshold; and
determining a velocity of one or more objects within the sample based on the respective positions of the imaging device corresponding to the two images with a level of correlation of the speckle pattern above a threshold.

While the above examples of the disclosure and optional features are described separately, it is to be understood that their provision in all possible combinations and permutations is contained within the disclosure. It is to be understood that various examples of the disclosure can comprise any or all of the features described in respect of other examples of the disclosure, and vice versa. Also, it is to be appreciated that any one or more or all of the features, in any combination, may be implemented by/comprised in/performable by an apparatus, a method, and/or computer program instructions as desired, and as appropriate.

### BRIEF DESCRIPTION

Some examples will now be described with reference to the accompanying drawings in which:
FIG. 1 shows an example device;
FIG. 2 shows an example method;
FIG. 3 shows another example device;
FIG. 4 shows an example device in use;
FIG. 5 shows another example; and
FIG. 6 shows an example apparatus.

The figures are not necessarily to scale. Certain features and views of the figures can be shown schematically or exaggerated in scale in the interest of clarity and conciseness. For example, the dimensions of some elements in the figures can be exaggerated relative to other elements to aid explication. Corresponding reference numerals are used in the figures to designate corresponding features. For clarity, all reference numerals are not necessarily displayed in all figures.

### DETAILED DESCRIPTION

Examples of the disclosure relate to determining velocities of objects that generate a speckle pattern when illuminated. The object could be blood within biological tissues or any other suitable object. Examples of the disclosure can use devices such as LiDAR systems to illuminate the sample and capture the images. This can enable the examples of the disclosure to be implemented in handheld devices such as mobile phones or any other suitable type of device. This can provide a convenient device for a user to use to measure their blood velocity or the velocity of any other suitable object.

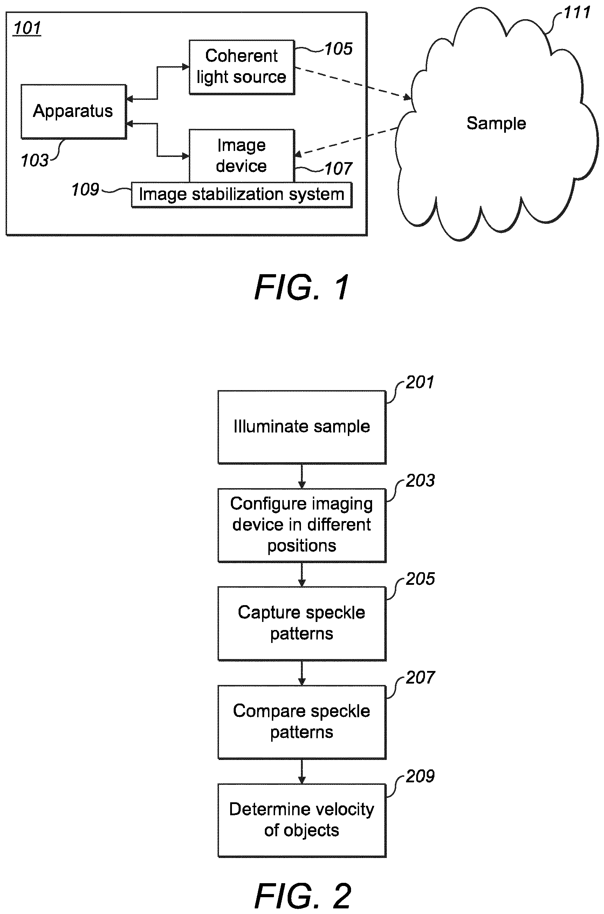

Fig. 1 schematically shows an example device 101 that could be used to implement examples of the disclosure.

The example device 101 could be a mobile phone, a camera, a health monitoring device, a computing device, a teleconferencing device, a virtual reality device, an augmented reality device or any other suitable type of device.

The example device 101 comprises an apparatus 103, a coherent light source 105, an imaging device 107, and an image stabilization system 109. Only components of the device 101 that are referred to in the following description are shown in Fig.1. Additional components could be provided in some examples of the disclosure. For instance, the device 101 could comprise a power source, a user interface or any other suitable components.

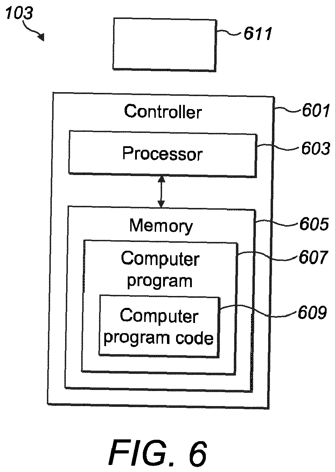

The apparatus 103 can comprise a controller comprising a processor and memory. Examples of an apparatus 103 are shown in Fig. 6. The apparatus 103 can be configured to enable control of the device 101. For example, the apparatus 103 can be configured to control the functions that are performed by the device 101.

The coherent light source 105 is configured to provide light for illuminating a sample 111. In some examples the coherent light source 105 can be configured to project a particular pattern on to the sample 111. The pattern can be configured to cause speckle images to be generated in light backscattered from the sample 111. The pattern that is projected by the coherent light source 105 could be a dot pattern or any other suitable type of pattern.

The coherent light source 105 can be configured to provide light at any suitable wavelength. The wavelength that is used could be determined by the sample 111 that is being monitored. For example, if the sample 111 comprises biological tissue the wavelength that is used could be in the infrared range because this will be absorbed by the biological sample. Other wavelengths of light could be used for different types of samples.

In some examples the coherent light source 105 could comprise a laser. The laser could be part of a LiDAR system or any other suitable system.

The imaging device 107 can comprise any means that can be configured to capture images. The imaging device 107 can comprise one or more image sensors which can be configured to convert light incident on the image sensor into an electrical signal to enable an image to be produced. The image sensors can be configured to detect light in the same wavelength range as is provided by the coherent light source 105. This can enable the imaging device 107 to be used to detect light backscattered from the sample 111. The image sensors can comprise, for example, digital image sensors such as charge-coupled-devices (CCD) or complementary metal-oxide-semiconductors (CMOS). The images that are obtained can comprise a speckle pattern.

The imaging device 107 can also comprises an optical arrangement. The optical arrangement comprises one or more optical components. The optical components can comprise means for focusing incident light onto the image sensor. The optical components can comprise one or more lenses. In some examples the lenses could be meta lenses or any other suitable type optical components.

In Fig. 1 the coherent light source 105 and the imaging device 107 are shown as separate components. In some examples they could be part of the same components. For example, the electronic device 101 could comprises a LiDAR system and the LiDAR system can comprise both a coherent light source 105 and an imaging device 107 for capturing LiDAR images. Other arrangements of the coherent light source 105 and the imaging device 107 could be used in other examples.

The image stabilization system 109 comprises means for stabilizing images captured by the imaging device 107. The image stabilization system 109 can comprise a mechanical system. The image stabilization system 109 is configured to move the imaging device 107, or parts of the imaging device 107. The image stabilization system 109 can be configured to move both the image sensor and the optical arrangement of the imaging device 107.

The image stabilization system 109 can comprise any suitable means for moving the respective components of the imaging device 107. In some examples the means could comprise electromagnets positioned around the housing of the imaging device 107. The electromagnets can be configured to be controlled in response to detected movement of the electronic device 101. This can enable images to be stabilized to take into account shaking or other movements of the electronic device 101.

In examples of the disclosure the image stabilization system 109 is configured to be controlled by the apparatus 103. This can enable the position of the imaging device 107 to be adjusted to track objects in the sample 111. This can enable a velocity of the tracked objects to be determined.

In the example of Fig. 1 the electronic device 101 is being used to monitor a sample 111. In some examples the electronic device 101 is configured to monitor the velocity of one or more objects within the sample 111. The one or more objects could be any objects that scatter the incident light from the coherent light sources 105 so as to create a speckle pattern. In some examples the object that is monitored could be the sample 111 itself.

Examples of objects that could be monitored could be a fluid such as blood. As blood moves through biological tissue it causes fluctuations in speckle patterns. These fluctuations give information about the movement and velocity of the blood through the biological tissue. Other movement of a biological sample, or part of a biological sample could be monitored in other examples, for instance, the fluctuations in the speckle pattern could be caused by movement of skin or tendons. In other examples the objects could be fluid moving through the respective components of a plant. Other types of samples 111 and objects could be used in other examples.

Fig. 2 shows an example method that could be implemented using examples of the disclosure. The method could be implemented using the example electronic device 101 shown in Fig. 1 and/or any other suitable device.

At block 201 the method comprises controlling a coherent light source 105 to illuminate a sample 111. The apparatus 103 of the electronic device 101 could be configured to control the coherent light source 105 so as to provide light suitable for generating a detectable speckle pattern when it is backscattered by the sample 111.

The apparatus 101 can be configured to control the wavelength of light that is used to illuminate the sample 111 so as cause the generation of a detectable speckle pattern. The wavelength of light that is used can be dependent upon the sample 111 that is being monitored. For instance, if the sample 111 comprises a biological sample then the light could comprise infrared light.

In some examples the coherent light source 105 can be configured to illuminate the sample 111 using a pattern of light. The pattern of light that is used can be selected so as to improve the resolution of the speckle patterns in the images. The pattern of light could be a dot pattern. The dot pattern can comprise a regular array of equally, or substantially equally, sized dots. Other patterns could be used in other examples.

The speckle pattern can comprise a granular pattern that can be observed in light backscattered from the sample 111. The speckle pattern can be random. The speckle pattern arises from phase differences caused by the objects in the sample 111. These phase differences cause interference within the backscattered light and this causes a spatially varying intensity distribution.

The sample 111 that is illuminated can comprise any sample 111 that comprises objects that produce a fluctuating speckle pattern as they move. In some examples the sample 111 can comprise objects that move in a linear or laminar motion. In some examples the sample 111 could comprise a biological sample. The biological sample could comprise an in-vivo or living sample 111. The objects that are monitored in the biological sample could comprise blood. Blood cells can generate fluctuating speckle patterns as it moves through tissues of the biological sample. The blood that is monitored could comprise venous flow, arterial flow, flow within tissues, microvasculature blood flow or any other type of blood flow. Examples of the disclosure can be used where the blood flow is laminar, or substantially laminar, over the scale of movement that can be tracked by the movement of the imaging device 107 controlled by the image stabilization system 109.

In some examples the objects that are monitored could comprise solid objects such as skin or tendons that can flex or deform as they are moved. Other biological samples could comprise plants. For instance, the sample 111 could comprise a phloem of a plant that is transporting sucrose and amino acids or other suitable objects. In some examples the sample 111 could comprise a non-biological sample. For instance, any channel that is transporting scattering objects and is, at least partially transparent, to the light from the coherent light source 105 could be monitored.

At block 203 the method comprises configuring the imaging device 107 into a plurality of different positions. The different positions can be relative to the sample 111. This can comprise moving the imaging device 107. In examples of the disclosure the apparatus 103 can be configured to control the image stabilization system 109 to control the movement of the imaging device 107. The movement is controlled so that the position of the imaging device 107 relative to the sample 111 is known at the times respective images are captured. The image stabilization system 109 can be controlled to make small fast movements of the imaging device 107. The image stabilization system 109 can be controlled to move the imaging device 107 at different speeds.

The image stabilization system 109 can be configured to move the whole of the imaging device 107 or just part of the imaging device 107. For instance, the image stabilization system 109 could be configure to move the image sensor and/or the optical arrangements. Therefore, moving the imaging device 107 could comprise moving the whole of the imaging device 107 or just part of the imaging device 107.

The movements of the imaging device 107 can keep the imaging device 107 in the same plane relative to the sample 111. The image stabilization system 109 can be configured to enable movement within the plane but restrict movement out of the plane.

An apparatus 103 can be configured to control the image stabilization system 109 to move the imaging device 107 to a plurality of different positions. In some examples this can comprise a start position at which a first image can be captured and one or more other position. In some examples the apparatus 103 can control the image stabilization system 109 to follow a movement protocol. The movement protocol can comprise a predefined sequence of movements. The predefined sequences can comprise different angular positions and translational positions for the imaging device 107. The movement protocol can comprise timing information that indicates a time at which the imaging device 107 should be in the respective positions. The movement protocol can comprise information relating to the speed at which the imaging device 107 should move between respective positions.

In some examples the movement protocol can be adjusted to enable the image stabilization system 109 to perform dual functions. That is, the image stabilization system 109 can be configured both to stabilize the images and to move the imaging device 107 to track the objects at the same time. To enable this the movements used in the movement protocol can be adapted to account for the movements needed for image stabilization. This means that the same movement protocol could result in different actual movements at different times depending upon the movement of the electronic device 101.

In some examples the movement protocol or other sequence of movements that are used to control the movement of the imaging device 107 can be adapted to take into account a known trajectory of the one or more objects that are being monitored. For example, if it is known that the one or more objects are moving horizontally in the sample 111 the movements of the imaging device 107 can be predominantly horizontal.

At block 205 the method comprises controlling the imaging device 107 to capture images of the sample 111 at the plurality of different positions. The imaging device 107 can be controlled to capture a plurality of different images with the imaging device 107 at a plurality of different positions. The images that are captured comprise the backscattered light from the sample 111 and so the images comprise speckle patterns.

At block 207 the method comprises comparing the speckle patterns. The apparatus 103 can be configured to compare speckle patterns in two or more different images.

The different images can comprise images that are captured with the imaging device 107 at different positions.

The comparison of the speckle patterns can comprise measuring a level of correlation of the speckle patterns in the respective images. The comparison can identify two images with a level of correlation above a threshold. In some examples the comparison can identity the two images, out of the respective pairs of images, with the highest level of correlation. In this case the threshold would be that the correlation is above the level of correlation in the other pairs of images. In some examples the comparison can identify two images that have a correlation level above a fixed or predetermined threshold.

If there is a low level of correlation between the speckle patterns in the images this can indicate that the scattering objects within the images are different. In this example a low level of correlation between the images would indicate that the objects that cause the scattering and generate the speckle pattern are different. This indicates that the movement of the imaging device 107 between the positions at which the respective images were captured is not synchronized, or not very well synchronized, with the movement of the objects in the samples 111.

If there is a high level of correlation between the speckle patterns in the images this can indicate that the scattering objects within the images are the same or similar. In this example a high level of correlation between the images would indicate that the movement of the imaging device 107 between the positions at which the respective images were captured is synchronized, or substantially synchronized with the movement of the objects in the samples 111.

In some examples the level of correlation in the speckle patterns between respective images could be identified by comparing the entirety of the respective images. In other examples just a subset of the respective images could be compared. In such examples a region of interest within the respective images could be identified and then a subset of the image comprised within the region of interest could be compared.

At block 209 the method comprises determining a velocity of one or more objects within the sample 111 based on the respective positions of the imaging device 107 corresponding to the two images with a level of correlation above a threshold.

The respective positions and times that the images identified at block 209 were captured can be determined. These positions and times can be converted into a velocity of the one or more objects 111. The velocity can be an absolute velocity.

Examples of the disclosure therefore enable a velocity of an object to be measured by using the correlation of respective images to find a movement of the imaging device 107 that tracks, or substantially tracks, the movement of the object. Such examples can be implemented using a LiDAR system and imaging devices 107 such as those found in mobile phones. As these examples can be used to monitor blood flow this can provide a useful and convenient health monitoring device for a user. Other devices could be used for other purposes in examples of the disclosure.

In some examples the speed of the objects that can be monitored can be limited by the rate at which the image stabilization system 109 can move the imaging device 107. For instance, the speeds could be limited to a range of 0.01-20cm/s. This range would cover the velocity of blood in human tissues and vessels. Blood within tissues would usually be expected to have a velocity of 0- 0.1cm/s and blood within vessels would be expected to have a velocity of 1.5-20cm/s.

In some examples the velocity of the objects might not be uniform. For example, the velocity of blood changes during a pulse cycle. In such cases the velocity that is calculated can be calculated as the distance between the image capture locations within a plane divided by the time between the image capture times.

In examples where the movement of the object is known not to be uniform the movement of the imaging device 107 could by synchronized with known, or estimated, changes in the movement of the object. For example, if the object comprises blood then the pulse rate of the sample 111 could be determined from a pulse monitoring device or any other suitable means. For example, from electrocardiogram data, from detecting changes in colour in the sample 111 or from any other suitable source. In such cases, to capture peak velocity the capturing of the images can be timed so as to coincide with the peak of the pulse waveform. In some examples different images captured at different parts of the pulse cycle could be captured so as to enable velocity at different times of the pulse could be monitored.

In some examples, in addition to comparing pairs of images, one or more reference images can be used. The reference image could be captured at an origin point or any other suitable location. The same location could be used for the same reference image for each pair of images. The reference image can be used to determine a component of the speckle pattern that is due to blood volume. This component could then be subtracted from the images that are compared to each other so that this component does not contribute towards the levels of correlation.

In some examples reference image can also be used to remove static speckle artefacts. The speckle artefacts could be caused by components of the sample 111 other than the objects that are being monitored. For example, the speckle artefacts could be caused by skin, vessel walls and other tissues which lie above the blood vessels.

The use of the reference image can improve the signal to noise ratio in the images that are compared. In some examples the reference image could also be used to adjust for movement of the electronic device 101 during the capture times of the images.

In some examples reference images at different locations could be obtained. This can enable the static speckle artefacts that could be caused by skin, vessel walls and other tissues which lie above the blood vessels to be accounted for in other locations.

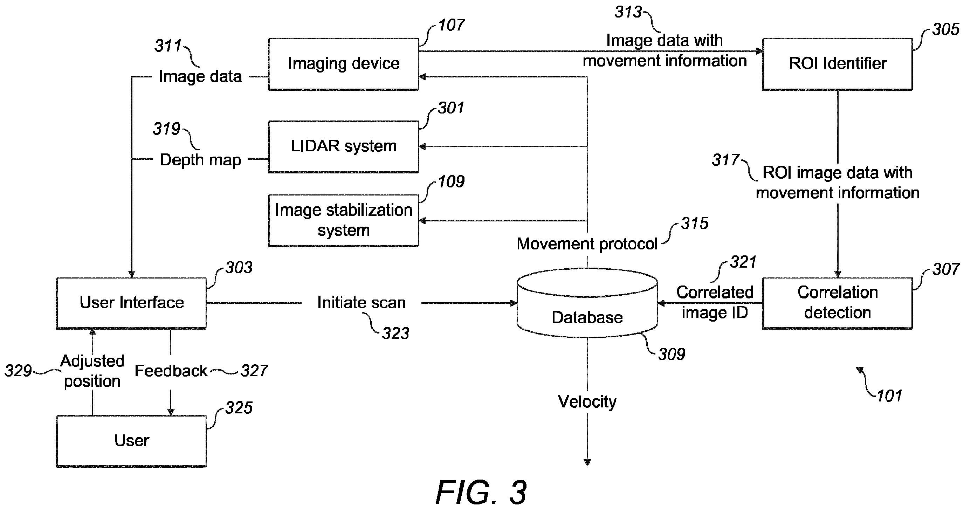

Fig. 3 schematically shows another example device 101 that could be used to implement examples of the disclosure. The example device 101 of Fig. 3 could be a specific implementation of an example device 101 as shown in Fig.1. Corresponding reference numerals are used for corresponding features.

In this example the electronic device 101 comprises an imaging device 107, a LiDAR system 301, an image stabilization system 109, a user interface 303, a region of interest (ROI) identifier module 305, a correlation detection module 307 and a database 309. In some examples the ROI identifier module 305, correlation detection module 307 and database 309 (or a combination of any one or more of these components) could be provided by an apparatus 103 as shown in Fig. 1.

The imaging device 107 can be as shown in Fig. 1 and described above. The imaging device 107 can be configured to capture images of a sample 111. The sample 111 is not shown in Fig. 3. The imaging device 107 can comprise an image sensor and an optical arrangement.

The imaging device 107 can be configured to provide image data 311 as an output. The image data 311 can comprise any data indicative of the images or parts of images that have been detected by the imaging device 107. In the example of Fig. 3 the imaging device 107 is configured to provide the image data 311 as an output to a user interface 303. This can enable the image data 311 to be rendered as an image for a user 325 of the electronic device 101. The rendering of the image could help the user 325 to position the electronic device 101 in a suitable position for monitoring the sample 111.

The imaging device 107 can be configured to provide image data 311 as an output. The image data 311 can comprise any data indicative of the images or parts of images that have been detected by the imaging device 107. In the example of Fig. 3 the imaging device 107 is configured to provide the image data 311 as an output to a user interface 303. This can enable the image data 311 to be rendered as an image for a user 325 of the electronic device 101. The rendering of the image could help the user 325 to position the electronic device 101 in a suitable position for monitoring the sample 111.

The imaging device 107 can also be configured to provide combined image data and movement information 313 as an output. The combined image data and movement information 313 can comprise any data indicative of the images or parts of images that have been detected by the imaging device 107 and also information relating to the movement of the imaging device 107. The information relating to the movement of the imaging device 107 can comprise information relating to a movement protocol that has been used to control movement of the imaging device 107. In some examples the information relating to movement of the imaging device 107 can comprise information indicative of the respective positions of the imaging device 107 at the times when the images were captured. In the example of Fig. 3 the imaging device 107 is configured to provide the combined image data and movement information 313 as an output to an ROI identifier module 305. This can enable the ROI identifier module 305 to use the image data and the corresponding information about the positions of the imaging device 107 to determine a ROI for the respective images.

The imaging device 107 is also configured to receive an input from the database 309. In this case the input comprises a movement protocol 315. The movement protocol 315 comprises a sequence of movements that the imaging device 107 is to make. The movement protocol 315 can indicate a plurality of positions for the imaging device 107 and a timing for the imaging device 107 to be located into those positions. The movement protocol 315 can comprise instructions relating to the speed at which the imaging device 107 is to be moved between respective positions.

The information about the movement protocol can be used by the imaging device 107 to combine the image data 311 with the movement information. This enables the position at which the imaging device 107 was located, when an image was captured to be identified.

The database 309 can store a plurality of different movement protocols 315. In such cases an appropriate movement protocol 315 to use can be selected based on any relevant factor such as the type of sample 111 that is being monitored, known information about the movement of objects within the sample such as the trajectory or an expected velocity range, or any other suitable factors.

The movement protocol 315 is also provided as an input to the image stabilization system 109. The image stabilization system 109 can be as shown in Fig. 1 and described above. The image stabilization system 109 can be configured to control the movement of the imaging device 107 in accordance with the instructions of the movement protocol 315.

An example of a movement protocol 315 could be:
a) Capture first image at time t
b) Move image stabilization system 109 1mm in the positive direction on the y axis at speed 20mm/s
c) Capture second image at time t+0.1s
d) Return to origin
e) Capture third image at time t+0.2s
f) Move image stabilization system 109 1mm in the positive direction on the y axis and 0.1 mm on the x axis at speed 20mm/s
g) Capture forth image at time t+0.3s
h) Return to origin.

The movement protocol 315 can continue until a sufficient number of angles and speeds have been trialled.

The movement protocol 315 can enable the imaging device 107 to move at different speeds relative to the object that is being monitored. The different speeds can be achieved by controlling the imaging device to move different distances in the same time interval or by keeping the imaging device 107 moving at the same speed but by covering different distances between the images, or by any combination of these.

In some examples the image stabilization system 109 can be configured to adjust the instructions of the movement protocol to account for movement or shaking of the electronic device 101. This can enable stabilization of the images while still ensuring that the imaging device 107 is located in the correct positions for capturing the respective images as instructed in the movement protocol 315.

The movement protocol 315 is also provided from the database 309 to the LiDAR system 301 of the electronic device 101.

The LiDAR system 301 can be configured to perform LiDAR scanning. The LiDAR scanning can be used to determine distances between the electronic device 101 and one or more other objects. In some examples the LiDAR system 301 could be used for identification of the objects or any other suitable purpose.

The LiDAR system 301 can comprise a coherent light source 105 such as a laser. The laser of LiDAR system can be used to illuminate a sample 111 so as to generate the speckle patterns that can be detected in imaged captured by the imaging device 107.

The coherent light that is provided by the LiDAR system 301 can be provided in any suitable format. In some examples the coherent light could be provided in pattern. The pattern could comprise dots that are projected at fixed angles from the electronic device 101 or in any other suitable arrangement.

The coherent light that is provided by the LiDAR system 301 can be provided at a sufficient intensity such that the speckle pattern generated by the sample 111 can be resolved by the imaging device 107. The imaging device 107 could be a camera such as the type of camera that is used in mobile phones and other communication devices.

The LiDAR system 301 can comprise any suitable means that can be configured provide the coherent light. In some examples the LiDAR system 301 can comprise an optical phased array, a micromirror arrangement or any other suitable means for providing the coherent light in a suitable pattern.

In some examples the LiDAR system 301 can be configured to identify a suitable region of the sample 111 to illuminate with the coherent light. The LiDAR system 301 can be configured to use raster scanning and image analysis to locate a suitable region of the sample 111 to illuminate with the coherent light. Other means for identifying the suitable region could be used in other examples.

Other means or systems for providing the coherent light could be used in other examples. For instance, a dot projector of a smart device could be used.

In some examples the LiDAR system 301 can be configured to provide a depth map 319. The depth map 319 can provide information relating to the distance between the electronic device 101 and the sample 111. This information could be used to help position the electronic device 101 relative to the sample 111.

In the example of Fig. 3 the image data 311 is provided to a user interface 303. The depth map 319, or other relevant information obtained by the LiDAR system 301, can also be provided to the user interface 303.

The user interface 303 can enable any means for enabling a user to receive outputs from and/or provide inputs to, the electronic device 101. The user interface 303 can comprise output devices such as displays, loudspeakers, tactile output devices or any other suitable means. The user interface 303 can comprise input devices such as touch screens, microphones, motion detectors or any other suitable means. In some examples the user interface 303 can comprise components that are both inputs and outputs, for example a touch screen can be configured to display information to a user 325 and also detect inputs made by the user 325.

In examples of the disclosure the user interface 303 can be configured to help to guide a user 325 to position the electronic device 101 in a location and orientation to enable a sample 111 to be monitored. For instance, the user interface 303 can be configured to provide instructions to the user 325 to enable the user to position the electronic device 101 so that the coherent light illuminates a region of the sample comprising the objects that are to be monitored. The instructions can also help the user to position the electronic device 101 in an appropriate orientation. For example, it can be used to help the user 325 to position the electronic device 101 so that an x-y plane of the imaging device 107 is parallel, or substantially parallel with the movement of objects within the sample 111.

In the example of Fig. 3 the user interface 303 is configured to provide feedback 327 to a user 325. The feedback 327 can audio, visual or tactile instructions. The feedback 327 can indicate a position that the electronic device 101 should be held in. In some examples the feedback can provide an indication as to whether the electronic device 101 is in a suitable position and/or orientation or not. If the electronic device 101 is not in a suitable position and/or orientation then the feedback 327 could comprise instructions as to how the position and/or orientation of the electronic device 101 should be adjusted.

When the user 325 adjusts the position of the electronic device 101 then the adjusted position 329 is detected by the user interface 303. The adjusted position can be analysed to determine whether or not the adjusted position is suitable for monitoring the movement of objects in the sample 111.

Any suitable means and methods can be used to determine suitable positions and/or orientations for the electronic device 101 relative to the sample 111. In some examples object recognition algorithms could be used. These could be configured to obtain images from the imaging device 107 and compare these to ideal, or substantially ideal, images. For instance, the perspective of a sample 111 in an image could be determined and compared to an ideal perspective. In some examples images comprising the speckle pattern could be captured by the imaging device 107. These images could then be analysed to detect regions of the image in which movement of the objects could be detected. A suitable position for the electronic device 101 could then be determined as a position in which this region is captured. The depth map 319 from the LiDAR system 301 can be used to determine the orientation of the electronic device 101 relative to the sample 111. This can be used to determine whether or not the electronic device 101 is in a plane parallel to the movement of the objects in the sample 111 or not.

When it has been determined that the electronic device 101 is in a suitable position and orientation then the user interface 303, or other suitable means, can provide an initiate scan signal 323. The initiate scan signal 323 can control the electronic device 101 to begin the scanning of the sample 111. This can initiate the movement of the imaging device 107 using the image stabilization system 109 and the capturing of the images of the sample 111 at the different positions.

The imaging device 107 is configured to collect the image data 311 during the scan. The image data can be combined with information about the movement protocol 315 to generate combined image data and movement information 313. The combined image data and movement information 313 can be provided as an output from the imaging device 107. The combined image data and movement information 313 can be provided as an input to an ROI identifier module 305.

The ROI identifier module 305 can be configured to identify the regions of the captured images in which the speckle pattern can identified. In such cases the ROI identifier module 305 can comprise a speckle pattern detection algorithm or any other suitable means that can be configured to segment the captured image based on detected speckle patterns.

In some examples there can be a plurality of speckle patterns within an image. The different speckle patterns could be caused by different dots from the pattern of coherent light. In such cases one or more of the speckle patterns can be selected by the ROI identifier module 305. The selection of the speckle pattern can be based on, intensity of the detected signals, orientation of the electronic device 101 relative to the sample, levels of correlation between respective images, or any other suitable factor of combination of factors.

The ROI identifier module 305 provides ROI image data with movement information 317 as an output. The combined ROI image data and movement information 317 can comprise any data indicative of the parts of images that have been selected by the ROI identifier module 305 and also information relating to the movement of the imaging device 107. The information relating to movement of the imaging device 107 can comprise information indicative of the respective positions of the imaging device 107 at the times when the images were captured.

The combined ROI image data and movement information 317 is provided as an input to a correlation detection module 307. The correlation detection module 307 is configured to detect the degree of speckle decorrelation between pairs of images or between the regions of interest of pairs of images. Any suitable processes or algorithms can be used to determine the level of correlation between respective images. The correlation detection module 307 can be configured to detect pairs of images with a level of correlation above a threshold. In some examples the correlation detection module 307 can be configured to detect pairs of images with the available set of images that have the highest level of correlation.

The correlation detection module 307 is configured to provide correlated image identification 321 as an output. The correlated image identification 321 can provide an indication of one or more pairs of images with a level of correlation above a threshold. The correlated image identification 321 can also comprise information relating to the positions of the imaging device 107 when the respective images in the pair were captured.

The correlated image identification 321 can be used to determine the velocity of the objects in the sample 111. The respective positions that the imaging device 107 was in when the images where captured, and the relative timings of the capturing of the images can be used to determine a velocity of the scattering objects in the sample. The velocity can comprise both the speed and the direction of the objects.

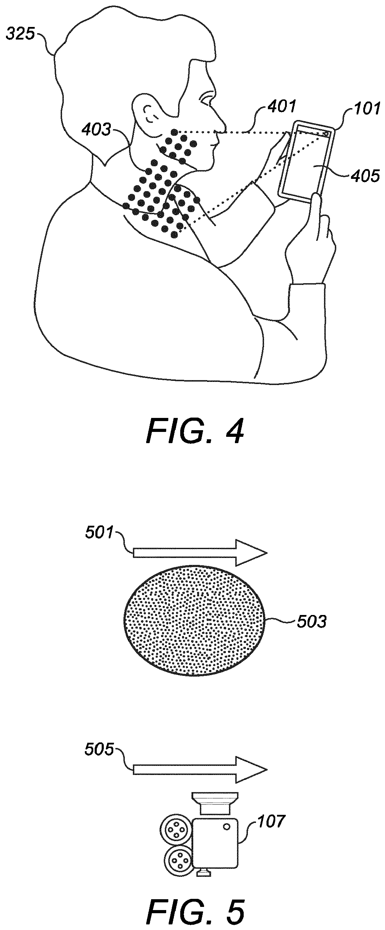

Fig. 4 shows an example of a user 325 using an electronic device 101 to monitor their own blood flow.

In this example the electronic device 101 is a mobile phone. Other types of electronic devices 101 could be used in other examples.

The user 325 is using the electronic device 101 to measure blood velocity in their neck. For example, the user 325 could be monitoring the blood velocity in the cardioid artery or through any other suitable tissues. In this case the sample 111 is the user's neck and the objects that are being monitored is the blood within the neck.

To enable their blood flow to be monitored the user 325 holds the electronic device 101 so that coherent light 401 from the coherent light source is incident on their neck. In this example the coherent light 401 comprises a dot pattern 403. Other patterns for the light could be used in other examples.

The electronic device 101 also comprises a display 405. The display 405 can be part of a user interface 303 that can be configured to provide instructions to the user 325 on how to position the electronic device 101. For example, the display 405 could be configured to provide visual cues to the user to indicate how to hold the electronic device 101 so that the dot pattern 403, or at least part of the dot pattern 403, is incident on their neck. The visual cues could comprise instructions as to whether or not the position of the electronic device 101 needs to be adjusted.

The visual cues can also instruct the user 325 to position the electronic device 101 a suitable distance away from their neck (or other sample 111). The suitable distance between the electronic device 101 and the sample 111 can be dependent upon the type of sample, the objects within the sample 111 that are being monitored, the intensity of the coherent light, the resolution of the imaging device 107, or any other suitable factors. In this example the electronic device 101 is being held approximately 1-5cm away from the user's neck. Other distances or ranges of distances could be used in other examples of the disclosure.

When it is detected that the electronic device 101 is in a suitable position the scanning of the user's neck can be performed by an imaging device 107. The imaging device 107 could be a camera of the electronic device 101. The scanning can comprise capturing images while the image stabilization system moves the imaging device 107 to a plurality of different positions.

Once the scanning has been completed a notification can be provided to the user 325. For instance, a notification could be displayed on the display indicating that the scan has been completed. This can inform the user 325 that they can move the device 101 from the scanning position.

Once the scan has been completed the images can be compared to find pairs of images with a high correlation and to determine the blood velocity from the respective positions of the imaging device 107 when the images were captured.

In the example of Fig. 4 the user 325 is monitoring their own blood flow. In this case the user 325 is both the user 325 of the electronic device 101 and the sample 111. In other cases a user 325 could use the electronic device 111 to monitor blood flow in a different person or in an animal or to measure the velocity of any other suitable objects.

Fig. 5 schematically shows how the movement of the imaging device 107 can be used to determine the velocity of the blood of other objects.

The object moves in a direction indicated by the arrow 501. The object can move with a velocity v. The object can move with a constant velocity or could have a varying velocity. For example, the real time velocity of blood can change during a pulse cycle.

A dot pattern 403 of coherent light is projected onto the blood. Scattering of the dot pattern by the blood generates the speckle pattern 503. The speckle pattern 503 changes as the blood flows though the tissues because the positions of the scatterers changes.

In examples of the disclosure an imaging device 107 is moved as indicted by the arrow 505.

If the movement of the imaging device 107 is not aligned with the movement of the blood then the positions of the blood cells relative to the imaging device 107 will be different. This would result in different speckle patterns in images captured by the imaging device 107. There would be low levels of correlation between pairs of images if the movement of the imaging device 107 is not aligned with the movement of the blood.

If the movement of the imaging device 107 is aligned with the movement of the blood then the positions of the blood cells relative to the imaging device 107 will be the same or similar. This would result in the same or similar speckle patterns in images captured by the imaging device 107. There would be high levels of correlation between pairs of images if the movement of the imaging device 107 is not aligned with the movement of the blood. Is this case the movement of the imaging device 107 tracks the movement of the blood.

Therefore, by finding images captured at different positions and times that have a high level of correlation between the speckle patterns the velocity of the blood can be determined.

Fig. 6 schematically illustrates an apparatus 103 that can be used to implement examples of the disclosure. In this example the apparatus 103 comprises a controller 601. The controller 601 can be a chip or a chip-set. In some examples the controller 601 can be provided within a communications device or any other suitable type of device.

In the example of Fig. 6 the implementation of the controller 601 can be as controller circuitry. In some examples the controller 601 can be implemented in hardware alone, have certain aspects in software including firmware alone or can be a combination of hardware and software (including firmware).

As illustrated in Fig. 6 the controller 601 can be implemented using instructions that enable hardware functionality, for example, by using executable instructions of a computer program 607 in a general-purpose or special-purpose processor 603 that may be stored on a computer readable storage medium (disk, memory etc.) to be executed by such a processor 603.

The processor 603 is configured to read from and write to the memory 605. The processor 603 can also comprise an output interface via which data and/or commands are output by the processor 603 and an input interface via which data and/or commands are input to the processor 603.

The memory 605 stores a computer program 607 comprising computer program instructions (computer program code) that controls the operation of the controller 601 when loaded into the processor 603. The computer program instructions, of the computer program 607, provide the logic and routines that enables the controller 601. to perform the methods illustrated in the accompanying Figs. The processor 603 by reading the memory 605 is able to load and execute the computer program 607.

The apparatus 103 comprises:
at least one processor 603; and
at least one memory 605 including computer program code 609;
the at least one memory 605 and the computer program code 609 configured to, with the at least one processor 603, cause the apparatus 103 at least to perform:
   controlling a coherent light source to illuminate a sample to cause generation of speckle patterns in light backscattered from the sample;
   controlling an image stabilization system to configure an imaging device in a plurality of different positions;
   controlling the imaging device to capture images of the sample at the plurality of different positions wherein the images comprise the speckle patterns;
   comparing the speckle patterns in images captured with the imaging device at different positions to identify two images with a level of correlation of the speckle pattern above a threshold; and
   determining a velocity of one or more objects within the sample based on the respective positions of the imaging device corresponding to the two images with a level of correlation of the speckle pattern above a threshold.

The apparatus 103 comprises:
at least one processor 603]; and
at least one memory 605 including computer program code 609;
the at least one memory 605 storing instructions that, when executed by the at least one processor 603, cause the apparatus 103 at least to:
   controlling a coherent light source to illuminate a sample to cause generation of speckle patterns in light backscattered from the sample;
   controlling an image stabilization system to configure an imaging device in a plurality of different positions;
   controlling the imaging device to capture images of the sample at the plurality of different positions wherein the images comprise the speckle patterns;
   comparing the speckle patterns in images captured with the imaging device at different positions to identify two images with a level of correlation of the speckle pattern above a threshold; and
   determining a velocity of one or more objects within the sample based on the respective positions of the imaging device corresponding to the two images with a level of correlation of the speckle pattern above a threshold.

As illustrated in Fig. 6, the computer program 607 can arrive at the controller 601 via any suitable delivery mechanism 611. The delivery mechanism 611 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid-state memory, an article of manufacture that comprises or tangibly embodies the computer program 607. The delivery mechanism can be a signal configured to reliably transfer the computer program 607. The controller 601 can propagate or transmit the computer program 607 as a computer data signal. In some examples the computer program 607 can be transmitted to the controller 601 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 607 comprises computer program instructions for causing an apparatus to perform at least the following or for performing at least the following:
controlling a coherent light source to illuminate a sample to cause generation of speckle patterns in light backscattered from the sample;
controlling an image stabilization system to configure an imaging device in a plurality of different positions;
controlling the imaging device to capture images of the sample at the plurality of different positions wherein the images comprise the speckle patterns;
comparing the speckle patterns in images captured with the imaging device at different positions to identify two images with a level of correlation of the speckle pattern above a threshold; and
determining a velocity of one or more objects within the sample based on the respective positions of the imaging device corresponding to the two images with a level of correlation of the speckle pattern above a threshold.

The computer program instructions can be comprised in a computer program 607, a non-transitory computer readable medium, a computer program product, a machine readable medium. In some but not necessarily all examples, the computer program instructions can be distributed over more than one computer program 607.

Although the memory 605 is illustrated as a single component/circuitry it can be implemented as one or more separate components/circuitry some or all of which can be integrated/removable and/or can provide permanent/semi-permanent/ dynamic/cached storage.

Although the processor 603 is illustrated as a single component/circuitry it can be implemented as one or more separate components/circuitry some or all of which can be integrated/removable. The processor 603 can be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory or memories that work together to cause an apparatus, such as a mobile phone or server, to perform various functions and
(c) hardware circuit(s) and or processor(s), such as a microprocessor(s) or a portion of a microprocessor(s), that requires software (for example, firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in Fig. 2 can represent steps in a method and/or sections of code in the computer program 607. The illustration of a particular order to the blocks does not necessarily imply that there is a required or preferred order for the blocks and the order and arrangement of the blocks can be varied. Furthermore, it can be possible for some blocks to be omitted.

The apparatus 103 can be provided in an electronic device, for example, a mobile terminal, according to an example of the present disclosure. It should be understood, however, that a mobile terminal is merely illustrative of an electronic device that would benefit from examples of implementations of the present disclosure and, therefore, should not be taken to limit the scope of the present disclosure to the same. While in certain implementation examples, the apparatus can be provided in a mobile terminal, other types of electronic devices, such as, but not limited to: mobile communication devices, hand portable electronic devices, wearable computing devices, portable digital assistants (PDAs), pagers, mobile computers, desktop computers, televisions, gaming devices, laptop computers, cameras, video recorders, GPS devices and other types of electronic systems, can readily employ examples of the present disclosure. Furthermore, devices can readily employ examples of the present disclosure regardless of their intent to provide mobility.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, the wording 'connect', 'couple' and 'communication' and their derivatives mean operationally connected/coupled/in communication. It should be appreciated that any number or combination of intervening components can exist (including no intervening components), i.e., so as to provide direct or indirect connection/coupling/communication. Any such intervening components can include hardware and/or software components.

As used herein, the term "determine/determining" (and grammatical variants thereof) can include, not least: calculating, computing, processing, deriving, measuring, investigating, identifying, looking up (for example, looking up in a table, a database or another data structure), ascertaining and the like. Also, "determining" can include receiving (for example, receiving information), accessing (for example, accessing data in a memory), obtaining and the like. Also, " determine/determining" can include resolving, selecting, choosing, establishing, and the like.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a', 'an' or 'the' is used in this document with an inclusive not an exclusive meaning. That is any reference to X comprising a/an/the Y indicates that X may comprise only one Y or may comprise more than one Y unless the context clearly indicates the contrary. If it is intended to use 'a', 'an' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

The above description describes some examples of the present disclosure however those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description of the examples of the present disclosure.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
controlling a coherent light source to illuminate a sample to cause generation of speckle patterns in light backscattered from the sample;
controlling an image stabilization system to configure an imaging device in a plurality of different positions;
controlling the imaging device to capture images of the sample at the plurality of different positions wherein the images comprise the speckle patterns;
comparing the speckle patterns in images captured with the imaging device at different positions to identify two images with a level of correlation of the speckle pattern above a threshold; and
determining a velocity of one or more objects within the sample based on the respective positions of the imaging device corresponding to the two images with a level of correlation of the speckle pattern above a threshold.

2. An apparatus as claimed in claim 1 wherein the apparatus comprises a LiDAR system and the images comprising the speckle patterns are LiDAR images.

3. An apparatus as claimed in any preceding claim wherein the coherent light source is configured to project a dot pattern onto the sample.

4. An apparatus as claimed in any preceding claim wherein the coherent light source is configured to use infrared light to illuminate the sample.

5. An apparatus as claimed in any preceding claim wherein the sample comprises a biological sample.

6. An apparatus as claimed in claim 5 where the one or more objects whose velocity is determined comprises blood within the biological sample.

7. An apparatus as claimed in claim 6 wherein the means are for obtaining information relating to a pulse and adjusting the movement of the imaging device to account for the pulse.

8. An apparatus as claimed in any preceding claim wherein the different positions of the imaging device comprise a plurality of different positions relative to the sample.

9. An apparatus as claimed in any preceding claim wherein the image stabilization system is controlled such that the positions of the imaging device are restricted to take into account a known direction of movement of the one or more objects.

10. An apparatus as claimed in any preceding claim wherein the image stabilization system is configured to move an image sensor and an optical arrangement of the imaging device.

11. An apparatus as claimed in any preceding claim wherein the level of correlation is compared for a subset of the image.

12. A device comprising an apparatus as claimed in any preceding claim wherein the device is at least one of: a telephone, a camera, a computing device, a teleconferencing device, a virtual reality device, an augmented reality device, a headset.

13. A method comprising:
controlling a coherent light source to illuminate a sample to cause generation of speckle patterns in light backscattered from the sample;
controlling an image stabilization system to configure an imaging device in a plurality of different positions;
controlling the imaging device to capture images of the sample at the plurality of different positions wherein the images comprise the speckle patterns;
comparing the speckle patterns in images captured with the imaging device at different positions to identify two images with a level of correlation of the speckle pattern above a threshold; and
determining a velocity of one or more objects within the sample based on the respective positions of the imaging device corresponding to the two images with a level of correlation of the speckle pattern above a threshold.

14. A method as claimed in claim 13 wherein the images comprising the speckle patterns are LiDAR images.

15. A computer program comprising computer program instructions that, when executed by processing circuitry, cause:
controlling a coherent light source to illuminate a sample to cause generation of speckle patterns in light backscattered from the sample;
controlling an image stabilization system to configure an imaging device in a plurality of different positions;
controlling the imaging device to capture images of the sample at the plurality of different positions wherein the images comprise the speckle patterns;
comparing the speckle patterns in images captured with the imaging device at different positions to identify two images with a level of correlation of the speckle pattern above a threshold; and
determining a velocity of one or more objects within the sample based on the respective positions of the imaging device corresponding to the two images with a level of correlation of the speckle pattern above a threshold.
